# EUROPEAN PATENT APPLICATION

(11) **EP 4 653 872 A1**
(43) Date of publication of application: **26.11.2025**
(21) Application number: 24382541.1
(22) Date of filing: 22.05.2024
(51) Int. Cl.: G01N 33/92

(54) **A SET OF BIOMARKERS AND AN IN VITRO METHOD FOR THE DIAGNOSIS OF METABOLIC DYSFUNCTION-ASSOCIATED STEATOHEPATITIS (MASH)**

(71) Applicant: Richart Jurado, Cristóbal Manuel, 43002 Tarragona (ES); Bertran Ramos, Laia, 43141 Vilallonga del Camp (ES)
(72) Inventor: Richart Jurado, Cristóbal Manuel, 43002 Tarragona (ES); Bertran Ramos, Laia, 43141 Vilallonga del Camp (ES)
(74) Representative: Ponti & Partners, S.L.P

(57) **Abstract**

The present invention relates to a set of biomarkers and an *in vitro* method for the diagnosis of metabolic dysfunction-associated steatohepatitis (MASH) in a non-alcoholic human being already diagnosed with metabolic dysfunction-associated steatotic liver disease (MASLD).

## Description

### Field of the invention

The present invention relates to a set of biomarkers and an *in vitro* method for the diagnosis of metabolic dysfunction-associated steatohepatitis (MASH), corresponding exactly to the previous known term in the art of non-alcoholic steatohepatitis (NASH), in non-alcoholic human beings already diagnosed with metabolic dysfunction-associated steatotic liver disease (MASLD), corresponding exactly to the previous known term in the art of non-alcoholic fatty liver disease (NAFLD).

### Background

Non-alcoholic fatty liver disease (NAFLD) is the term for a range of conditions caused by a build-up of fat in the liver in absence of alcohol consumption. This disease has a global prevalence of close to 30% but is more common in people who are overweight or obese. Nevertheless, recently, a multi-society Delphi consensus statement suggested replacing the commonly known term NAFLD with Metabolic Dysfunction-Associated Steatotic Liver Disease (MASLD) to reduce stigmatization. MASLD is defined by the presence of hepatic steatosis (accumulation of fat in more than 5% of hepatocytes), identified through imaging or biopsy, combined with at least one metabolic alteration related to 1) increased body mass index (BMI) or waist circumference, 2) high glucose or glycated haemoglobin A1c (HbA1c) levels, 3) raised up blood pressure or 4) deregulated lipid profile (triglycerides or high-density lipoprotein cholesterol (HDL-C)) **[1].**

The spectrum of MASLD is broad and can range from Metabolic Dysfunction-Associated Steatotic Liver (MASL) which is simple steatosis and generally nonprogressive, to the progressive form Metabolic Dysfunction-Associated Steatohepatitis (MASH), formerly known as Non-alcoholic Steatohepatitis (NASH). MASH represents the severe condition of MASLD, characterized by histological features such as lobular inflammation and hepatocyte ballooning. MASH carries an increased risk of progressing to fibrosis, cirrhosis, or hepatocellular carcinoma. Although liver biopsy is the gold standard for diagnosing and staging MASH, its invasiveness may not be suitable for all individuals. Therefore, it is essential to identify non-invasive biomarkers for accurate diagnosis of MASLD subjects already diagnosed with MASH. Furthermore, previously reported metabolomic and lipidomic studies have identified metabolites involved in key metabolic pathways in MASLD and MASH pathogenesis, including alterations in amino acid metabolism, and primarily in lipid metabolism **[2,3].** Recent lipidomic studies have reported alterations in fatty acid, ceramide and phospholipid metabolism, suggesting induction of MASH through mitochondrial dysfunction, oxidation and inflammation **[4].** However, these lipid metabolite signatures require validation in other cohorts.

Accordingly, the problem to be solved by the present invention is to find a suitable *in vitro* diagnosis method in order to properly diagnose subjects with Metabolic Dysfunction-Associated Steatohepatitis (MASH), formerly known as Non-alcoholic Steatohepatitis (NASH), which have already been diagnosed with Metabolic Dysfunction-Associated Steatotic Liver Disease (MASLD), formerly known as Non-alcoholic Fatty Liver Disease (NAFLD).

The present invention addresses this problem by providing a novel set of blood circulating lipid metabolite biomarkers.

### Summary of the invention

In a first aspect, the present invention relates to a set of biomarkers comprising at least the following blood circulating lipid metabolites:
- deoxycholic acid;
- oxylipin 9-HODE;
- phosphatidylcholines PC 34:2, PC 36:2, and PC 36:3;
- phosphoethanolamines PE O-36:6 and PE P-34:3;
- lysophosphatidylinositol LPI 16:0; and
- diacylglycerol DG 36:0.
for the diagnosis *in vitro* of non-alcoholic steatohepatitis in a non-alcoholic human being already diagnosed with metabolic dysfunction-associated steatotic liver disease.

In a second aspect, the present invention relates to an in vitro method for diagnosing non-alcoholic steatohepatitis in a non-alcoholic human being already diagnosed with metabolic dysfunction-associated steatotic liver disease.

### Detailed description

In a first aspect, the present invention relates to a set of biomarkers comprising at least the following blood circulating lipid metabolites:
- deoxycholic acid;
- lipoxin 9-HODE;
- phosphatidylcholines PC 34:2, PC 36:2, and PC 36:3;
- phosphoethanolamines PE O-36:6 and PE P-34:3;
- lysophosphatidylinositol LPI 16:0; and
- diacylglycerol DG 36:0.
for the diagnosis *in vitro* of MASH in a non-alcoholic human being already diagnosed with MASLD.

In the context of the present invention, the term "patient" refers to the non-alcoholic human being already diagnosed with MASLD.

In the context of the present invention, the terms "Non-alcoholic Fatty Liver Disease" or "NAFLD" or "Metabolic Dysfunction-Associated Steatotic Liver Disease" or "MASLD" are interchangeable used and represents the presence of hepatic steatosis along with at least one cardiometabolic alteration.

In the context of the present invention, the terms "Non-alcoholic Steatohepatitis" or "NASH" or "Metabolic Dysfunction-Associated Steatohepatitis" or "MASH" are interchangeable used and represents the presence of hepatic steatosis in more than 5% of hepatocytes joined to hepatocyte ballooning and lobular inflammation with or without liver fibrosis, along with at least one cardiometabolic alteration.

It is also very important to note that the "human being" in the context of the present invention is a human being or patient diagnosed with MASLD but he or she is a "non-alcoholic" human, being defined as a human being with an alcohol consumption lower than 20 g per day in women and 30 g per day in men **[1],** because recurrent alcohol consumption would interfere with a proper diagnosis and stratification. If the patient is a moderate or severe alcoholic or usually consumes alcohol, he/she will be not accepted in the diagnosis of the present invention because alcohol also causes fatty liver (Alcoholic Steatotic Liver Disease (ASLD)) and alcoholic hepatitis. The applicability would only be for DIAGNOSIS in "non-alcoholic" patients.

**"Deoxycholic acid"** is one of the secondary bile acids, which are metabolic byproducts of intestinal bacteria. Deoxycholic acid has been used since its discovery in various fields of human medicine. In the human body, deoxycholic acid is used in the emulsification of fats for absorption in the intestine.

**"9-HODE"** is an oxylipin, a bioactive lipid generated by the oxidation of polyunsaturated fatty acids. Oxylipins are both potent and short lived. Therefore, they are not stored; rather, they are synthesized *de novo* and regulated closely and exert their effect in a paracrine or autocrine manner. Linoleic acid (LA) is also metabolized through hydroxylation by CYP epoxygenases to form hydroxyl-LA metabolites known as hydroxyoctadecadienoic acids (HODEs). HODEs are associated with oxidative stress, and the 9-HODE was described as pro-inflammatory.

**"Phosphatidylcholines"** or **"PC"** are a class of phospholipids that incorporate choline as a headgroup. They are a major component of biological membranes and can be easily obtained from a variety of readily available sources, such as egg yolk or soybeans, from which they are mechanically or chemically extracted using hexane. Chemically, they are a 1,2-diacyl-sn-glycero-3-phosphocholine. According to the established nomenclature referred to PC, the first number represents the total of carbon atoms in the acyl groups at C-1 and C-2 and the second figure represents the number of double bounds. Accordingly, PC 34:2 represents that the acyl groups at C-1 and C-2 contain 34 carbons in total with two double bonds, PC 36:2 represents that the acyl groups at C-1 and C-2 contain 36 carbons in total with two double bonds and PC 36:3 represents that the acyl groups at C-1 and C-2 contain 36 carbons in total with three double bonds.

**"Phosphoethanolamines"** or **"Phosphatidylethanolamines"** or **"PE"** is an ethanolamine derivative that is used to construct two different categories of phospholipids. One category termed a glycerophospholipid and the other a sphingomyelin, or more specifically within the sphingomyelin class, a sphingophospholipid. PE is a polyprotic acid with two pKa values at 5.61 and 10.39. In particular PE-O 36:6 represents 1-(1Z-hexadecenyl)-2-(5Z,8Z,11Z,14Z,17Z-eicosapentaenoyl)-glycero-3-phosphoethanolamine and PE-P34:3 represents (2-aminoethoxy)[(2R)-3-[(1Z)-hexadec-1-en-1-yloxy]-2-[(6Z,9Z,12Z)-octadeca-6,9,12-trienoyloxy]propoxy]phosphinic acid.

**"Lysophosphatidylinositol"** or **"LPI"** or **"lysoPI"** or **"L-α-lysophosphatidylinositol"** is an endogenous lysophospholipid and endocannabinoid neurotransmitter. LPI is a bioactive lipid generated by phospholipase A family of lipases which is believed to play an important role in several diseases. LPI 16:0 represents 1-palmitoyl-2-hydroxy-sn-glycero-3-phospho-inositol.

**"Diacylglycerol"** or **"diglyceride"** or **"DG"** or **"DAG"** is a glyceride consisting of two fatty acid chains covalently bonded to a glycerol molecule through ester linkages. DG 36:0 represents 1,2-dioctadecanoyl-sn-glycerol.

In another aspect, the present invention relates to an *in vitro* method for diagnosing MASH in a non-alcoholic human being already diagnosed with MASLD comprising:
- determining the amount of the biomarkers as defined in the first aspect of the invention in a biological sample of a non-alcoholic human being already diagnosed with MASLD;
- comparing the amount of the biomarkers as defined in the first aspect of the invention in the biological sample to a corresponding amount of the same biomarker found in a biological sample from one or more comparable non-alcoholic human beings who have been already diagnosed with MASLD and only present simple steatosis (no inflammation, ballooning or fibrosis);
- diagnosing that the non-alcoholic human being already diagnosed with MASLD is susceptible to or suffering from MASH if the amounts of deoxycholic acid, 9-HODE, phosphatidylcholines PC 34:2, PC 36:2 and PC 36:3, posphoethanolamines PE O-36:6 and PE P-34:3, and lysophosphatidylinositol LPI 16:0 are increased with respect to the amount of the same corresponding biomarker found in the biological sample from the one or more comparable non-alcoholic human beings who have been already diagnosed with MASLD and only present simple steatosis (no inflammation, ballooning or fibrosis); and the amount of diacylglycerol DG 36:0 is lowered with respect to the amount of the same diacylglycerol DG 36:0 found in the biological sample from the one or more comparable non-alcoholic human beings who have been already diagnosed with MASLD and only present simple steatosis (no inflammation, ballooning or fibrosis).

As used in the present invention, "simple steatosis", means hepatic steatosis without inflammation, ballooning or fibrosis.

The biological sample as used in the present invention relates to a sample already isolated from the human being to be used *in vitro.* The set of biomarkers as defined in the first aspect of the invention are also isolated from the sample for the diagnosis.

In a preferred embodiment, the biological sample used in the *in vitro* method of the invention is a serum sample.

In another preferred embodiment, the human being is a woman. More preferably, said woman is a woman with morbid obesity. Even more preferably, said woman with morbid obesity is a non-menopausal woman.

In this disclosure and in the claims, terms such as "comprises", "comprising", "containing" and "having" are open-ended terms and can mean "includes", "including" and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an" and "the" include plural referents unless context clearly indicates otherwise. Similarly, the word "or" is intended to include "and" unless the context clearly indicate otherwise.

It should be noted that the term "approximately" or "about" applied to the values used earlier and later in this document includes a margin of error of ± 5 %, such as, for example, ± 4 %, ± 3%, ± 2 %, ± 1 %.

It is noted that any of the embodiments disclosed herein can be taken alone or combined with any other embodiment disclosed herein unless the context specifies otherwise. In other words, for example, a preferred option of a defined feature can be combined with a more or less preferred option of another feature.

The invention will be now illustrated with the following examples, which do not intend to limit its scope.

### EXAMPLE

Given the emergence of metabolomics and lipidomics as relevant techniques for characterizing the metabolic profile in MASLD, our previous untargeted metabolomic analysis in serum samples of morbidly obese women with liver histological diagnoses focused on identifying potential metabolic factors involved in MASLD/MASH pathogenesis for use as new non-invasive biomarkers. The study specifically found lipid metabolites significantly characteristic of MASLD and MASH subjects **[5].**

In this context, our LC/MS-based untargeted lipidomic assay aims to identify the lipid metabolite profile of MASH subjects in a well-characterized cohort of morbidly obese women with hepatic histological diagnoses. The aim is to identify those lipid factors involved in MASH pathogenesis that could serve as diagnostic or predictive non-invasive biomarkers for MASH.

### 1. Subjects

In this comprehensive investigation, we conducted an untargeted lipidomics analysis using liquid chromatography coupled to a mass spectrometer (LC/MS) on serum samples obtained from a cohort of 216 women with morbid obesity (BMI ≥ 40 kg/m2) that were slated for laparoscopic bariatric surgery.

To ensure a homogeneous subject cohort and mitigate the impact of confounding variables, particularly sex-related differences, our study exclusively focused on women. This approach is rooted in the well-established understanding that men and women exhibit significant disparities in body composition, energy balance and hormonal profiles. Moreover, prior research has also underscored sex-specific variations in lipid and glucose metabolism.

Ethical considerations were paramount throughout the study, with approval obtained from the institutional review board at Institut Investigació Sanitària Pere Virgili (IISPV, CElm (23c/2015)). Written informed consent was diligently obtained from all participants. Exclusion criteria were applied to eliminate potential confounders, such as individuals with acute illnesses, acute or chronic inflammatory or infective diseases, and those with end-stage malignant disease. Furthermore, menopausal women and those undergoing contraceptive treatment were also excluded from the study. In addition, women with an alcohol intake exceeding 20g per day and recurrent smokers have been excluded from this study.

### 2. Liver Histological Diagnosis

Liver samples were procured during laparoscopic bariatric surgery, in a formaldehyde solution. Subsequently, biopsies were subjected to evaluation and classification by a proficient hepatopathologist using eosin-hematoxylin staining. In this regard, women with morbid obesity and corresponding histological diagnoses were first stratified into two distinct categories: normal liver (NL, n = 44) and MASLD (n = 172) groups.

Within the MASLD cohort, further subgroups were delineated based on Kleiner et al.'s criteria: Simple Steatosis (SS, n = 66) and MASH (n = 106). Notably, all instances of MASH were confined to grades I-II, exhibiting mild to moderate inflammation without the presence of hepatic fibrosis, aligning with stage F0 on the Ishak scoring system.

### 3. Anthropometrical and Biochemical Variables

The anthropometric assessment encompassed the measurement of weight, height, waist-hip ratio and BMI calculation.

Specialized nurses conducted blood extraction using a BD Vacutainer^{®} system, following an overnight fasting period just before the scheduled bariatric surgery. Venous blood samples were collected in ethylenediaminetetraacetic acid tubes, subsequently separated into plasma and serum aliquots through centrifugation (3500 rpm, 4 °C, 15 min), and then stored at -80°C until processing. Biochemical analyses included parameters such as glucose, insulin, HbA1c, total cholesterol, HDL-C, low density lipoprotein-cholesterol (LDL-C), triglycerides, aspartateaminotransferase (AST), alaninoaminotransferase (ALT), gamma-glutamyl transferase (GGT), alkaline phosphatase (ALP), lactate dehydrogenase (LDH) and ferritin levels. These analyses were conducted utilizing a conventional automated analyzer.

### 4. LC/MS Methods

### Samples Preparation for the LC/MS Analysis:

Serum metabolites were extracted in 12µL of isopropanol and vortex-mixing for 10 seconds, incubated at 4 °C for 30 min and centrifuged (at 12000 rpm for 10 min at 4 °C).

### LC/MS Setup:

LC/MS was performed with a Thermo Scientific Vanquish Horizon Ultra High Performance LC system interfaced with a Thermo Scientific Orbitrap ID-X Tribrid Mass Spectrometer (Thermo Scientific, Waltham, MA, USA).

Metabolites were separated by reverse-phase chromatography with an Acquity Ultra Performance LC C18-RP (ACQUITY UPLC BEH C18 1.7µM, Waters). Mobile phase A was acetonitrile/water (60:40) (10mM ammonium formate), and mobile phase B was isopropanol/acetonitrile (90:10) (10mM ammonium formate). Solvent modifiers were used to enhance ionization and to improve the LC resolution in positive and negative ionization mode.

Separation was conducted under the following gradient: 0-2 min, 15-30% B; 2-2.5 min, 48% B; 2.5-11 min, 82% B; 11-11.5 min, 99% B; 11.5-12 min, 99% B; 12-12.1 min, 15% B; 12.1-15 min, 15% B.)

For MS detection, heated electrospray ionization settings were set in positive and negative ionization modes as follows: source voltage, 3.5 kV (positive), 2.8 kV (negative); ion transfer tube, 300 °C; vaporizer temperature, 300 °C; sheath gas (N2) flow rate, 50 a.u.; auxiliary gas (N2) flow rate, 10 a.u.; sweep gas (N2) flow rate, 1 a.u.; s-lens rf level, 60%; SCAN-mode; resolution, 120000 (at m/z 200); AGC target, 50%; maximum injection time, 200ms.

Quality control samples consisting of pooled samples from each condition were injected at the beginning and periodically through the workflow.

MS/MS acquisition was performed at a resolution of 15000, and the normalized collision energy of the HCD cell was stepped at 10-20-30-40%. This collision energy was tested to obtain appropriate precursor ion and product intensities. The quadrupole isolation window was 1 m/z. The maximum injection time of the C-trap was customized for each inclusion list. Xcalibur 4.4 software (Thermo Scientific, Waltham, MA, USA) was used for LC/MS instrument control and data processing.

### LC/MS Data Analysis:

Thermo raw data files were transformed to .mzML using Proteowizard's MSconvert. Then, mzML files were processed using RHermes, a computational tool that improves the selectivity and sensitivity for comprehensive metabolite profiling and identification. RHermes substitutes the conventional untargeted metabolomics workflow that detects and annotates peaks, for an inverse approach that directly interrogates raw LC/MS data points using a comprehensive list of unique molecular formulas that were retrieved from large compound-centric databases (e.g., HMDB, ChEBI, NORMAN). They are used to generate a large set of ionic formulas by combining metabolite molecular formulas with expected adduct ions depending on the polarity. RHermes solves the limitations of peak detection by finding series of scans, named SOI (Scans Of Interest), which are defined as clusters of data points that match an ionic formula and are concentrated within a short period of time. Identification was performed by HERMES using two strategies: cosine spectral matching using an in-house DB, containing MS/MS spectra from MassBankEU, MoNA, HMBD, Riken and NIST14 databases, and using MassFrontier version 8.0 SR1 (Thermo Scientific, Waltham, MA, USA) matching against the mzCloud database. Spectral hits with high similarity scores (>0.8) were manually revised to assess correct metabolite identifications.

SOls are qualitative elements that are suited for metabolite annotation and identification. Analytically though, only well-behaved chromatographic peaks are to be quantified, these peaks have a sharp elution profile and a high signal-to-noise ratio. In order to perform SOI quantification, SOI scan series are evaluated and partitioned into well-behaved peaks in order to extract accurate abundance values. This is performed using the qHermes R package that applies the Centwave algorithm originally found in the XCMS R package. Centwave algorithm determines the baseline and boundaries of well-behaved peaks (according to a set of parameters) and assigns an abundance value using the apex (highest value) of the peak, these boundaries may be different from SOI retention time ranges.

After data quality is assured and corrections have been performed, the data is ready for statistical testing.

### Lipid Compound Identification:

To elucidate the identity of SOI and assign that to features, we have performed a set of fragmentation spectra using LC/MS that physically split the molecular structure into a reproducible and recognizable pattern. These patterns can be compared to those found in reference metabolite databases and matched using similarity analysis. In here, we have used 2 different databases to obtain such similarity: HERMES and MS2ID.

In the case of lipidomics, lipid structures are well-characterized and metabolite spectra can be assigned to a lipid family. This is achieved by the LipidMS R package.

Some databases such as Lipidmaps may simplify lipid naming in a coded manor. Chain notations in phospholipids or glycerolipids may also be noted as X:Y, X being the number of carbons of the fatty acid and Y the number of insaturations.

### 5. Statistical Analysis

Group differences were assessed using the nonparametric Mann-Whitney test (for biochemical and anthropometric variables) or one-way ANOVA (for lipidomics data). Statistical significance was set at p-values < 0.05.

Only significant ions quantified in more than 80% of the samples underwent statistical testing for differences across experimental groups using one-way ANOVA. The obtained statistical results were subjected to adjustment through the false discovery rate (FDR) p-value correction method. Fold-changes, or ratios, were calculated by dividing the mean value of an experimental group by that of a reference group, particularly in case-control studies. These ratios, represented by log2-ratios of the means of the A/B groups, serve to filter or rank preliminary statistical results. A fold-change greater than 0 indicates higher intensity in group A compared to group B, and viceversa. To streamline the identification process, Log2FC and FOR-adjusted p-value thresholds were fine-tuned. The intention was to manage the number of metabolite entities for manual review, making the process feasible. Detailed data on the concentration of significant lipid metabolites, expressed in terms of Log2FC, mean, and standard deviation, can be found in Table 2.

### 6. Results

**Table 1. Lipid metabolites concentration increased or decreased in serum samples of patients with morbid obesity and MASH compared to subjects with morbid obesity and simple steatosis.**

| **Family** | | **Increased Levels** | **Decreased Levels** |
|---|---|---|---|
| Bile acids | | Deoxycholic acid | |
| Diacylglycerols (DG) | | | DG (36:0) |
| Fatty acids | | 9-HODE | |
| Phospholipids | Phosphatidylcholines (PC) | PC (34:2) | |
| | | PC (36:2) | |
| | | PC (36:3) | |
| | Phosphoethanolamines (PE) | PE (O-36:3) | |
| | | PE (P-34:3) | |
| | Lyso-phosphatidylinositol (LPI) | LPI (16:0) | |

*One-way ANOVA test was used to identify significant differences. Data on concentration in log fold change (FC) and p-values, as well as the mean and standard deviation of each lipid specie, are given in Table 2.*

**Table 2. Data from significant lipid metabolites concentration expressed in the Log2FC, mean and SD. One-way ANOVA test was used to identify significant differences between groups (MASH and simple steatosis (SS)) (adjusted p-value <0.05 was considered significant).**

| **Name** | **log2FC (MASH vs SS)** | **MEAN_MASH** | **SD_MASH** | **MEAN_SS** | **SD_SS** | **adjusted p-value** |
|---|---|---|---|---|---|---|
| Deoxycholic acid | 0,74 | 62085,75 | 68618,58 | 37168,86 | 35587,95 | 1.761e-02 |
| DG (36:0) | -0,85 | 11288475,2 | 14342783 | 20394420,7 | 13774778,9 | 1.493e-02 |
| LPI (16:0) | 0.88 | 96934,28 | 146096,04 | 52811,32 | 50672,53 | 4.735e-02 |
| 9-HODE | 0,83 | 1491368,34 | 1789676.5 | 841067,75 | 1356733,63 | 2.981e-02 |
| PE (P-34:3) | 0,61 | 1003601,7 | 720823,87 | 658389,8 | 460175,2 | 8.220e-03 |
| PE (O-36:3) | 0,61 | 7190795,64 | 5611204,96 | 4700030,05 | 3490213,44 | 3.367e-02 |
| PC (34:2) | 1,55 | 2142283,93 | 4639767,82 | 730078,48 | 2178442,18 | 3.570e-02 |
| PC (36:3) | 1,41 | 543494,57 | 1145259,04 | 204837,8 | 584955,82 | 4.461e-02 |
| PC (36:2) | 1,68 | 957558,67 | 2155816,33 | 298501,29 | 926440,43 | 2.526e-02 |

To conclude, in this LC/MS-based untargeted lipidomics study, meticulously conducted on a homogeneous cohort of fertile women with morbid obesity, our investigation has reported a set of increased lipid metabolites such as the oxylipin 9-HODE, some PC, PE and the LPI (16:0), and decreased levels of the DG (36:0), that seems to be discriminatory in MASH subjects compared to individuals with simple steatosis. Thus, this panel of lipid metabolites could be used as a non-invasive diagnostic tool for MASH.

### References

**[1]** Rinella ME, Lazarus JV, Ratziu V, et al. A multisociety Delphi consensus statement on new fatty liver disease nomenclature. J Hepatol. 2023;79(6):1542-1556. doi:10.1016/j.jhep.2023.06.003
**[2]** Kim HY. Recent advances in nonalcoholic fatty liver disease metabolomics. Clin Mol Hepatol. 2021;27(4):553-559. doi:10.3350/cmh.2021.0127
**[3]** Masoodi M, Gastaldelli A, Hyötyläinen T, et al. Metabolomics and lipidomics in NAFLD: biomarkers and non-invasive diagnostic tests. Nat Rev Gastroenterol Hepatol. 2021;18(12):835-856. doi:10.1038/s41575-021-00502-9
**[4]** Béland-Bonenfant S, Rouland A, Petit JM, Vergès B. Concise review of lipidomics in nonalcoholic fatty liver disease. Diabetes Metab. 2023;49(3):101432. doi:10.1016/j.diabet.2023.101432
**[5]** Bertran L, Capellades J, Abelló S, et al. LC/MS-Based Untargeted Metabolomics Study in Women with Nonalcoholic Steatohepatitis Associated with Morbid Obesity. Int J Mol Sci. 2023;24(12):9789. Published 2023 Jun 6. doi:10.3390/ijms24129789

## Claims

1. A set of biomarkers comprising at least the following blood circulating lipid metabolites:
- deoxycholic acid;
- oxylipin 9-HODE;
- phosphatidylcholines PC 34:2, PC 36:2, and PC 36:3;
- phosphoethanolamines PE O-36:6 and PE P-34:3;
- lysophosphatidylinositol LPI 16:0; and
- diacylglycerol DG 36:0.
for the diagnosis *in vitro* of metabolic dysfunction-associated steatohepatitis (MASH) in a non-alcoholic human being already diagnosed with metabolic dysfunction-associated steatotic liver disease (MASLD).

2. An *in vitro* method for diagnosing metabolic dysfunction-associated steatohepatitis (MASH) in a non-alcoholic human being already diagnosed with metabolic dysfunction-associated steatotic liver disease (MASLD) comprising:
- determining the amount of the biomarkers as defined in claim 1 in a biological sample of a non-alcoholic human being already diagnosed with MASLD;
- comparing the amount of the biomarkers as defined in claim 1 in the biological sample to a corresponding amount of the same biomarker found in a biological sample from one or more comparable non-alcoholic human beings who have been already diagnosed with MASLD and only present simple steatosis;
- diagnosing that the non-alcoholic human being already diagnosed with MASLD is susceptible to or suffering from MASH if the amounts of deoxycholic acid, 9-HODE, phosphatidylcholines PC 34:2, PC 36:2 and PC 36:3, posphoethanolamines PE O-36:6 and PE P-34:3, and lysophosphatidylinositol LPI 16:0 are increased with respect to the amount of the same corresponding biomarker found in the biological sample from the one or more comparable non-alcoholic human beings who have been already diagnosed with MASLD and only present simple steatosis; and the amount of diacylglycerol DG 36:0 is lowered with respect to the amount of the same diacylglycerol DG 36:0 found in the biological sample from the one or more comparable non-alcoholic human beings who have been already diagnosed with MASLD and only present simple steatosis.

3. The *in vitro* method according to claim 2 wherein the biological sample is a serum sample.

4. The set of biomarkers or the *in vitro* method according to any of claims 1 to 3, wherein the human being is a woman.

5. The set of biomarkers or the in vitro method according to claim 4, wherein said woman is a woman with morbid obesity.

6. The set of biomarkers or the in vitro method according to claim 5, wherein said woman is a non-menopausal woman with morbid obesity.
